# EUROPEAN PATENT APPLICATION

(11) **EP 1 308 152 A1**
(43) Date of publication of application: **07.05.2003**
(21) Application number: 01125924.9
(22) Date of filing: 30.10.2001
(51) Int. Cl.: A61K 7/16, A61K 7/18

(54) **Single-component dental composition for dentin desensitisation**

(71) Applicant: Italmed S.N.C. Di Galli G. & Pacini G., 50132 Firenze (IT)
(72) Inventor: Galli, Giovanna, c/o Italmed S.n.c., 50132 Firenze (IT); Pacini, Gigliola, c/o Italmed S.n.c., 50132 Firenze (IT)
(74) Representative: Celestino, Marco

(57) **Abstract**

A dental composition for treatment of dentinal hypersensitivity. It comprises at least an insoluble micronised fluorine salt, with crystals size less than 5 microns, and at least a soluble potassium salt. This way, the effect of mechanical obliteration is carried out by the insoluble micronised fluorine salt that penetrates the tubular orifices, whereas the depolarising action of the nervous fibres is carried out by the soluble potassium salt.

## Description

### Field of the invention

The present invention relates to dentistry and more precisely it relates to a gel composition treatment of the dentinal hypersensitivity.

The invention relates to also a method for making this composition.

### Brief description of the prior art

Dentinal hypersensitivity is a problem often found by dentists in their patients and is often intensified when eating hot or cold food, sweet or acidulous food, as well as when brushing teeth.

In particular, a dentist tests a dentinal exposition as hypersensitivity after stimulation with air blow. To reduce such hypersensitivity the dentist applies a desensitising composition directly on the exposed dentin, for example when treating deep cavities, when replacing dental layers, when desensitising stumps before placing dental prosthesis. At the end of the effect of any anaesthetic previously given to the patient, the preliminary application of such products reduces or eliminates the pain. It is therefore felt the need of a desensitising composition whose effect is sufficient and lasts with time.

Among the known desensitising compositions, a two-component product described in W09944570, in the name of the same applicant and incorporated by reference in the present description, comprises two different solutions or two different gels to be applied in succession on the exposed dentin. The solutions or gels comprise two soluble potassium salts, as first solution or as first gel, and a soluble calcium plus a soluble strontium salt as second solution or as second gel.

It is also known, in US4990327, a two-component product wherein a first phase contains strontium ions and a second phase contains fluorine ions. When mixing the two phases, strontium fluoride is formed directly on the exposed dentin, whereas a direct application on the teeth of strontium fluoride already formed is said to be not much effective.

Even if the above cited products are effective, the need is also felt of a single-component product, which does not need to be mixed with another component just before or during the application, and which is effective like the two-component products.

### Summary of the invention

It is therefore object of the present invention to provide a single-component composition, used for dentinal desensitisation, which has an effective capacity of mechanical obliteration of the dentinal tubules in addition to a capacity of depolarisation of the dentinal nervous fibres, allowing a high effectiveness and a long duration, as well as a practical use.

According to a first aspect of the invention, this object is achieved by a dental composition, whose characteristic is that it comprises at least an insoluble micronised fluorine salt, with crystals size less than 5 microns, and at least a soluble potassium salt.

This way, the effect of mechanical obliteration is carried out by the insoluble micronised fluorine salt that penetrates the tubular orifices, whereas the depolarising action on the nervous fibres is carried out by the two soluble potassium salt.

A possible range of particles sizes of the insoluble fluorine salt is the following:
- 40% less than 1,5 microns,
- 40% set between 1,5 and 2,5 microns,
- 20% more than 2,5 microns and less than 5 microns.

Preferably, the insoluble micronised fluorine salt is chosen among strontium fluoride, barium fluoride, zinc fluoride, magnesium fluoride or a mixture of at least two of them.

To the insoluble micronised fluorine salt a further insoluble micronised salt can be added chosen among:
strontium oxalate, barium oxalate, zinc oxalate, magnesium oxalate.

As soluble potassium salt, it can be chosen among potassium chloride, potassium fluoride or a mixture thereof.

According to another aspect of the invention, a method for making a single-component desensitising composition comprises the steps of:
- preparing a first solution of potassium fluoride and a second solution with a salt capable of reacting with the potassium fluoride thus forming an insoluble fluorine salt and a soluble potassium salt;
- mixing the two solutions that immediately lead to a micronised precipitate (colloidal or in any case micro-crystalline) consisting in the insoluble fluorine salt and in the soluble potassium salt;
- maintaining the state of micronised precipitate of the insoluble fluorine salt with the addition, immediately after the reaction, of a gelling agent.

By using strontium chloride as second solution, after the union a product of reaction is obtained formed by:
- said micronised precipitate consisting in the strontium fluoride,
- a solution resulting from double exchange reaction, containing possible excess potassium fluoride and potassium chloride.

Advantageously, the gelling agent is Carboxy Methyl Cellulose (CMC), which is added under stirring immediately after the reaction (for example within a minute). This way, owing to the CMC the growth is avoided of the strontium fluoride crystals, which thus remain at the state of micro crystals and in part are at the colloidal state.

The second solution can advantageously comprise a preserver, in species sodium benzoate.

According to a preferred embodiment, using strontium chloride and zinc chloride (or magnesium chloride or barium chloride) as second solution together with the sodium benzoate, a similar result is obtained, with two insoluble fluorine salts; the strontium fluoride and the zinc fluoride (or magnesium fluoride or barium fluoride) have a crystals size maintained in the range of micro crystals in the same way as above.

A different embodiment of the method provides the steps of:
- preparing strontium fluoride in powder;
- preparing a solution of potassium fluoride and potassium chloride;
- mixing with water and CMC thus making a micronised gel by a colloid mill.

A possible general weight proportion of the composition is the following :

| | | |
|---|---|---|
| first solution | potassium fluoride | 5-20% |
| second solution | strontium chloride | 5-15% |
| | sodium benzoate | 0,1-0,5% |
| gelling agent | sodium CMC | 1-3% |
| solvent | deionised water | qb 100 ml |

A other possible general weight proportion of the composition is the following:

| | | |
|---|---|---|
| first solution | potassium fluoride 5-20% | |
| second solution | strontium chloride 5-15% | |
| | zinc chloride | 1-10% |
| | (or magnesium chloride chloride) | or barium |
| | sodium benzoate | 0,1-0,5% |
| gelling agent | sodium CMC | 1-3% |
| solvent | deionised water | qb 100 ml |

A further possible general weight proportion of the composition is the following :

| | | |
|---|---|---|
| first solution | potassium fluoride 5-15% | |
| | potassium oxalate 3-5% | |
| second solution | strontium chloride 5-15% | |
| | sodium benzoate | 0,1-0,5% |
| gelling agent | sodium CMC | 1-3% |
| solvent | deionised water | qb 100 ml |

In order to show better the composition according to the present invention, some examples are given hereinafter.

### EXAMPLE 1

For making a first composition according to the invention, the following have been mixed:
potassium fluoride 15%
strontium chloride 10%
sodium benzoate 0,2% (preserver)
sodium CMC 1,35%
deionised water73,45%

In the resulting gel the products of the equilibrium reactions are:

| products | generated moles | g in 1 Kg |
|---|---|---|
| SrF₂ | 0,375 | 47,107 |
| Cl- | 0,750 | 26,587 |
| F- | 1,831 | 34,789 |
| K+ | 2,581 | 100,97 |

or:

| products | generated moles | g in 1 Kg |
|---|---|---|
| SrF₂ | 0,375 | 47,107 |
| KCl | 0,750 | 55,912 |
| KF | 1,831 | 106,381 |

The desensitising gel has been packed in single-component 0,33 ml containers and has been tested on 112 patients (totally 198 treated teeth), of age between 19 and 56, 38 men and 74 women, having abrasions/erosions, not generated by decay processes, but caused by chemical or mechanical agents, with high hypersensitivity with blow of air. The patients had had one or more teeth with cervical dentin exposed since at least one week and sensitive especially to heat stimuli.

The application of the gel has revealed two mechanism of action based, on one hand, on the obliterating action of the insoluble micronised fluorine salt that occlude the tubular orifices (mechanical obliteration) and, on the other hand, on a contemporaneous depolarising action on the nervous fibres by the two soluble potassium salt.

### EXAMPLE 2

For making a second composition according to the invention, the following have been mixed:
potassium fluoride 11%
strontium chloride 8%
zinc chloride 4%
sodium benzoate 0,2%
sodium CMC 1,8%
deionised water75%
In the resulting gel the products of the equilibrium reactions are:

| products | generated moles | g in 1 Kg |
|---|---|---|
| SrF2 | 0,3 | 37,686 |
| ZnF2 | 0,293 | 30,296 |
| Cl- | 1,186 | 42,043 |
| F- | 0,707 | 13,433 |
| K+ | 1,893 | 74,016 |

or:

| products | generated moles | g in 1 Kg |
|---|---|---|
| SrF2 | 0,3 | 37,686 |
| ZnF2 | 0,293 | 30,296 |
| KCl | 1,186 | 88,416 |
| KF | 0,707 | 41,076 |

### EXAMPLE 3

By mixing:
potassium oxalate 5% e
potassium fluoride 10%
strontium chloride 10%
sodium benzoate 0,2%
sodium CMC 1,35%
deionised water73,45%
in the gel the following result: strontium oxalate, strontium fluoride, KF + KCl (or K+ Cl- F-)

### EXAMPLE 4

By mixing:
potassium fluoride 11%
strontium chloride 8%
barium chloride 4%
sodium benzoate 0,2%
sodium CMC 1,8%
deionised water75%
   in the gel the following result: strontium fluoride, barium fluoride, KF + KCl (or K+ Cl- F-)

### EXAMPLE 5

By mixing:
potassium fluoride 11%
strontium chloride 8%
magnesium chloride 4%
sodium benzoate 0,2%
sodium CMC 1,8%
deionised water75%
   in the gel the following result: strontium fluoride, magnesium fluoride, KF + KCl (or K+ Cl- F-)

### EXAMPLE 6

By mixing:
potassium oxalate 5%
potassium fluoride 10%, and
strontium chloride 7%
zinc chloride 3%
sodium benzoate 0,2%
sodium CMC 1,35%
deionised water73,45%
in the gel the following result: strontium oxalate, strontium fluoride, zinc fluoride, KF + KCl (or K+ Cl- F-)

### EXAMPLE 7

By mixing:
potassium oxalate 5%
potassium fluoride 10%, and
strontium chloride 7%
barium chloride 3%
sodium benzoate 0,2%
sodium CMC 1,35%
deionised water73,45%
In the gel the following result: strontium oxalate, strontium fluoride, barium oxalate, barium fluoride, KF + KCl (or K+ Cl- F-)

### EXAMPLE 8

By mixing:
potassium oxalate 5%
potassium fluoride 10%, and
strontium chloride 7%
magnesium chloride 3%
sodium benzoate 0,2%
sodium CMC 1,35%
deionised water73,45%
In the gel the following result: strontium oxalate, strontium fluoride, magnesium oxalate, magnesium fluoride, KF + KCl(or K+ Cl- F-)

The foregoing description of a specific embodiment will so fully reveal the invention according to the conceptual point of view, so that others, by applying current knowledge, will be able to modify and/or adapt for various applications such an embodiment without further research and without parting from the invention, and it is therefore to be understood that such adaptations and modifications will have to be considered as equivalent to the specific embodiment. The means and the materials to realise the different functions described herein could have a different nature without, for this reason, departing from the field of the invention. It is to be understood that the phraseology or terminology employed herein is for the purpose of description and not of limitation.

## Claims

1. A single-component dental composition for desensitisation of the exposed dentin, or for treating deep cavities, replacing dental layers, desensitising stumps before placing dental prosthesis, **characterised in that** it comprises at least an insoluble micronised fluorine salt, with crystals size less than 5 microns, and at least a soluble potassium salt.

2. Composition according to claim 1, wherein the particles sizes of the insoluble fluorine salt are:
- 40% less than 1,5 microns,
- 40% set between 1,5 and 2,5 microns,
- 20% higher of 2,5 microns and less than 5 microns.

3. Composition according to claim 1, wherein said insoluble micronised fluorine salt is chosen among strontium fluoride, barium fluoride, zinc fluoride, magnesium fluoride or a mixture of at least two of them.

4. Composition according to claim 1, wherein said at least one soluble potassium salt is chosen among: potassium chloride, potassium fluoride or a mixture of them.

5. Composition according to claim 1, wherein to said insoluble micronised fluorine salt a further insoluble micronised salt is added chosen among: strontium oxalate, barium oxalate, zinc oxalate, magnesium oxalate, or a mixture of at least two of them.

6. A method for making a single-component desensitising composition comprising the steps of:
- preparing a first solution of potassium fluoride
- preparing a second solution with a salt capable of reacting with the potassium fluoride and forming an insoluble fluorine salt and a soluble potassium salt for double exchange reaction,
- mixing the two solutions that immediately lead to a micronised precipitate, at the colloidal state or in any case micro-crystalline, consisting in said insoluble fluorine salt and in said soluble potassium salt;
- adding, immediately after the reaction, of a gelling agent that keeps the insoluble fluorine salt in the state of micronised precipitate within a resulting solution, containing possible excess potassium fluoride and the soluble potassium salt.

7. Method according to claim 6, wherein said gelling agent is Carboxy Methyl Cellulose (CMC), which is added under stirring immediately after the reaction, avoiding the growth of the crystals of strontium fluoride owing to said CMC, which thus remain at the state of microcrystals and in part at the colloidal state.

8. Method according to claims 6 or 7, wherein a general weight proportion of the composition is the following :
| | | |
|---|---|---|
| first solution | potassium fluoride | 5-20% |
| second solution | strontium chloride | 5-15% |
| | sodium benzoate | 0,1-0,5% |
| gelling agent | sodium CMC | 1-3% |
| solvent | deionised water | qb 100 ml |

9. Method according to claims 6 or 7, wherein a general weight proportion of the composition is the following :
| | | |
|---|---|---|
| first solution | potassium fluoride | 5-20% |
| second solution | strontium chloride | 5-15% |
| | zinc chloride | 1-10% |
| | (or magnesium chloride or barium chloride) | |
| preserver | sodium benzoate | 0,1-0,5% |
| gelling agent | sodium CMC | 1-3% |
| solvent | deionised water | qb 100 ml |

10. Method according to claims 6 or 7, wherein a general weight proportion of the composition is the following :
| | | |
|---|---|---|
| first solution | potassium fluoride 5-15% | |
| | potassium oxalate 3-5% | |
| second solution | strontium chloride 5-15% | |
| | sodium benzoate | 0,1-0,5% |
| gelling agent | sodium CMC | 1-3% |
| Solvent | deionised water | qb 100 ml |

11. A method for making a single-component desensitising composition comprising the steps of:
- preparing strontium fluoride in powder;
- preparing a solution of potassium fluoride and potassium chloride;
- mixing with water and CMC thus making a micronised gel by a colloid mill.
